# EUROPEAN PATENT SPECIFICATION

(11) **EP 3 486 654 B1**
(45) Date of publication and mention of the grant of the patent: **18.08.2021**
(21) Application number: 17827644.0
(22) Date of filing: 12.07.2017
(51) Int. Cl.: G01N 33/531, G01N 33/543, G01N 33/53, G01N 33/558

(54) **DETECTION METHOD USING IMMUNOCHROMATOGRAPHY**
DETEKTIONSVERFAHREN MIT VERWENDUNG VON IMMUNCHROMATOGRAPHIE
PROCÉDÉ DE DÉTECTION METTANT EN OEUVRE L'IMMUNOCHROMATOGRAPHIE

(30) Priority: 13.07.2016 JP 2016138888
(43) Date of publication of application: 22.05.2019
(73) Proprietor: Sekisui Medical Co., Ltd., Tokyo 103-0027 (JP)
(72) Inventor: NISHITANI, Kimiyoshi, Tokyo 103-0027 (JP); OCHIAI, Yasushi, Tokyo 103-0027 (JP)
(74) Representative: Hoffmann Eitle
(86) International application number: PCT/JP2017/025343
(87) International publication number: WO 2018/012517

(56) References cited:
- EP-A1- 2 338 989
- EP-A1- 2 829 879
- EP-A1- 2 833 141
- WO-A2-2013/132347
- JP-A- H03 148 065
- JP-A- 2001 289 852
- JP-A- 2003 014 768
- JP-A- 2013 195 403
- JP-A- 2013 205 408
- JP-A- 2013 246 064

## Description

### TECHNICAL FIELD

The present invention relates to a method of inhibiting nonspecific reaction in an immunochromatographic method of detecting a fecal sample (feces-derived sample), as well as a detection kit and a sample diluent used in the method.

### BACKGROUND ART

A detection method using immunochromatography is widely used because of high specificity and sensitivity as well as convenience. Examples of samples applied to the detection method include urine, feces, saliva, blood, serum, etc., which are suitable as clinical test samples and widely used as diagnostically useful samples.

Particularly, in order to use feces as a sample to detect an analyte contained therein by utilizing immunochromatography, it is necessary to eliminate as much as possible the influences of a substance interfering directly with development of the sample by immunochromatography and a substance causing a reaction as if an analyte is present despite the absence thereof, i.e., so-called nonspecific reaction.

Gelatin, casein, bovine serum albumin, synthetic polymers, and the like, are known as general blocking agents for eliminating nonspecific reaction, and methods of allowing these agents to coexist in a system of reaction using immunochromatography are known (Patent Documents 1 and 2).

However, it is unknown whether these agents actually have an effect on inhibition of nonspecific reaction caused by a fecal sample.

Patent Document 3 describes a problem in the case of using feces as a specimen that the detection by ELISA etc. using feces as a specimen have resulted in low sensitivity and have frequently caused nonspecific reaction, having made it difficult to judge the result. This document discloses a method of inhibiting nonspecific reaction by using a specimen diluent of pH 9.0 to 10.0 so as to solve this problem and detect norovirus or sapovirus by using immunochromatography. However, this method has a problem in terms of handling because it is necessary to prepare a sample diluent on the alkaline side, which is not common for sample diluents.

Patent Document 4 discloses a method of enhancing the detection sensitivity of an immunoreaction by pretreating a biological sample with a specimen pretreatment liquid containing an organic acid such as citric acid and a surfactant.

Although feces are included in a long list of biological samples, those confirmed in the examples are nasal discharge or throat swab of influenza patients, and the detection method is not a method using immunochromatography.

As described above, in a method of detecting an analyte in a fecal sample by using immunochromatography, a technique is not yet established for a method of inhibiting nonspecific reaction caused by a fecal sample.

Patent Document 5 (WO 2013/132347) relates to an improved immunoassay for S100A9 provides increased reproducibility and accuracy for the determination of calprotectin, allowing more accurate diagnosis and screening for inflammatory bowel disease.

Patent Document 6 (EP 2 338 989) relates the provision of a method for preparing a stool sample that enables nucleic acids in a stool to be stably preserved without requiring a complex procedure, by mixing a collected stool with the stool sample preparation solution having a water-soluble organic solvent containing an organic acid.

Patent Document 7 (EP 2 833 141 A1) relates to an immunoassay method which can measure an antigen with high sensitivity and accuracy carried out in the presence of a polycarboxylic acid type surfactant.

### CITATION LIST

### PATENT LITERATURE

Patent Document 1: Japanese Laid-Open Patent Publication No. 2003-344406
Patent Document 2: Japanese Laid-Open Patent Publication No. 2002-148266
Patent Document 3: Japanese Laid-Open Patent Publication No. 2004-301684
Patent Document 4: WO 02/010744
Patent Document 5: WO 2013/132347
Patent Document 6: EP 2 338 989
Patent Document 7: EP 2 833 141 A1

### SUMMARY OF INVENTION

### TECHNICAL PROBLEM

A problem to be solved by the present invention is to provide a method of inhibiting nonspecific reaction unique to feces of infants in an immunochromatographic detection method in which an infant fecal sample is developed in an insoluble membrane to detect an analyte in the sample.

### SOLUTION TO PROBLEM

The present invention provides an immunochromatographic method of detecting an analyte in a fecal sample of neonates, infants, and children under seven years old,
(I) comprising the step of:
   performing an immunoreaction in the presence of a compound having in the molecule two or three carboxy groups,
   or
(II) comprising the steps of:
   (A) supplying a sample diluent containing (i) a compound having in the molecule two or three carboxy groups and (ii) a buffer solution as well as a fecal sample to a sample-supply portion of a test strip,
      the test strip having
      a membrane made up of a porous body including at least the sample-supply portion, a developing portion, and a detecting portion, a part of the developing portion retaining in an elutable manner a conjugate containing a first antibody labeled with a labeling substance, and the detecting portion having a second antibody immobilized in a part of the developing portion on the downstream side of the conjugate-retaining part;
   (B) bringing the analyte in the sample into contact with the conjugate; and
   (C) detecting a complex between the analyte in the sample and the conjugate in the detecting portion,
      wherein said compound having in the molecule two or three carboxy groups is a dicarboxylic acid or a tricarboxylic acid.

The present invention also provides a method of inhibiting nonspecific reaction in an immunochromatographic method of detecting an analyte in a fecal sample of neonates, infants, and children under seven years old,
(I) comprising the step of:
   performing an immunoreaction in the presence of a compound having in the molecule two or three carboxy groups,
   or
(II) comprising the steps of:
   (A) supplying a sample diluent containing (i) a compound having in the molecule two or three carboxy groups and (ii) a buffer solution as well as a fecal sample to a sample-supply portion of a test strip,
      the test strip having a membrane made up of a porous body including at least the sample-supply portion, a developing portion, and a detecting portion, a part of the developing portion retaining in an elutable manner a conjugate containing a first antibody labeled with a labeling substance, the detecting portion having a second antibody immobilized in a part of the developing portion on the downstream side of the conjugate-retaining part;
   (B) bringing the analyte in the sample into contact with the conjugate; and
   (C) detecting a complex between the analyte in the sample and the conjugate in the detecting portion,
wherein said compound having in the molecule two or three carboxy groups is a dicarboxylic acid or a tricarboxylic acid.

The present invention provides the use of an immunochromatographic detection kit for detecting an analyte in a fecal sample of neonates, infants, and children (under seven years old), wherein the immunochromatographic detection kit comprises:
(1) an immunochromatographic test strip having a membrane made up of a porous body including at least a sample-supply portion, a developing portion, and a detecting portion, a part of the developing portion retaining in an elutable manner a conjugate containing a first antibody labeled with a labeling substance, the detecting portion having a second antibody immobilized in a part of the developing portion on the downstream side of the conjugate-retaining part; and
(2) a sample diluent containing a compound having in the molecule two or three carboxy groups and a buffer solution,
wherein said compound having in the molecule two or three carboxy groups is a dicarboxylic acid or a tricarboxylic acid.

The present invention also provides the use of a diluent for detecting an analyte in a fecal sample of neonates, infants, and children under seven years old in an immunoassay, wherein the diluent contains a compound having in the molecule two or three carboxy groups and a buffer solution, wherein said compound having in the molecule two or three carboxy groups is a dicarboxylic acid or a tricarboxylic acid.

The present invention provides a sample pretreatment method for inhibiting nonspecific reaction in immunoassay of a fecal sample of neonates, infants, and children under seven years old, comprising the step of bringing the fecal sample of neonates, infants, and children under seven years old into contact with a sample diluent containing (i) a compound having in the molecule two or three carboxy groups and (ii) a buffer solution, wherein said compound having in the molecule two or three carboxy groups is a dicarboxylic acid or a tricarboxylic acid.

As a result of extensive studies for solving the problem, the present inventors have found that nonspecific reaction unable to be inhibited by conventional methods can be inhibited for fecal samples of infants by using a sample diluent containing a compound having in the molecule a dicarboxylic acid or a tricarboxylic acid for the present invention, thereby completing the present invention.

### ADVANTAGEOUS EFFECTS OF INVENTION

By using a compound having in the molecule a dicarboxylic acid or a tricarboxylic acid for the present invention, nonspecific reaction unique to an infant fecal sample can be inhibited to accurately detect an analyte in the sample in a detection method using immunochromatography.

According to the present invention, unique nonspecific reaction unable to be inhibited by conventional methods can be inhibited for fecal samples of infants, and therefore, an analyte can accurately be detected in infant fecal samples by using immunochromatography.

### BRIEF DESCRIPTION OF DRAWINGS

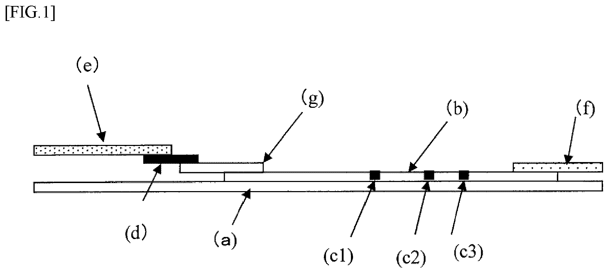

[Fig. 1] Fig. 1 is a schematic configuration diagram of a test strip of the present invention.

### DESCRIPTION OF EMBODIMENTS

### (Sample)

The present invention is directed to a sample derived from human feces of infants. The present invention produces an effect of inhibiting nonspecific reaction derived from feces of infants.

The term "infants" used in the present invention is intended to include all of neonates, infants, and children under seven years old. Feces may directly be used as a sample or may appropriately be diluted with a sample diluent before used as a sample. Feces may appropriately be diluted and filtered before being used as a sample. The sample diluent is sometimes also referred to as a specimen diluent, a sample extract, a sample developer, etc. which are all synonymously used.

### (Sample Diluent)

The sample diluent of the present invention contains at least a compound containing in the molecule a dicarboxylic acid or a tricarboxylic acid and preferably further contains a buffer solution. Examples of the compound containing in the molecule two or three carboxy groups include so-called dicarboxylic acids, such as oxalic acid (ethanedioic acid), malonic acid (propanedioic acid), succinic acid (butanedioic acid), glutaric acid (pentanedioic acid), adipic acid (hexanedioic acid), fumaric acid ((E)-But-2-enedioic acid), maleic acid ((Z)-But-2-enedioic acid), L-glutamic acid, and L-aspartic acid, and the compound is preferably maleic acid, L-aspartic acid, or glutamic acid.

Examples of a compound containing three carboxy groups in the molecule include tricarboxylic acids, such as aconitic acid (2-carboxyprop-1-enetricarboxylic acid), citric acid, isocitric acid, tricarballylic acid, and trimesic acid, and the compound is preferably citric acid.

In the present invention, a compound containing in the molecule two or three carboxy groups may be the compound itself as described above containing two or three carboxy groups in the molecule or may be a salt or a derivative of the compound. Examples of the salt include metal salts such as sodium salt, potassium salt, and calcium salt, and examples of the derivative include amide, ester, acid anhydride, and the like.

The compound containing in the molecule a dicarboxylic acid or a tricarboxylic acid in the sample diluent of the present invention is preferably in the concentration range of 5 mM to 300 mM.

When the number of the carboxy groups is two, the range is more preferably 10 mM to 300 mM, further preferably from 20 mM to 300 mM, furthermore preferably from 50 mM to 300 mM. Additionally, the range may be 30 mM to 300 mM, 40 mM to 300 mM, 60 mM to 300 mM, 70 mM to 300 mM, 80 mM to 300 mM, 90 mM to 300 mM, or 100 mM to 300 mM. The range may also be 10 mM to 250 mM, 10 mM to 200 mM, 10 mM to 150 mM, or 10 mM to 100 mM.

When the number of the carboxy groups is three, the range is more preferably 5 mM to 250 mM, further preferably 10 mM to 250 mM, furthermore preferably 20 mM to 250 mM. Additionally, the range may be 30 mM to 250 mM, 40 mM to 250 mM, 50 mM to 250 mM, 60 mM to 250 mM, 70 mM to 250 mM, 80 mM to 250 mM, 90 mM to 250 mM, or 100 mM to 250 mM. The range may also be 5 mM to 200 mM, 5 mM to 150 mM, or 5 mM to 100 mM.

Examples of the buffer solution contained in the sample diluent of the present invention include commonly used buffer solutions such as phosphate buffer, Tris buffer, and Good's buffer. The sample diluent of the present invention may be a diluent containing the compound and a buffer solution, may be a buffer solution containing the compound, or may further contain salts such as NaCl, a stabilizer such as sucrose and a preservative, and an antiseptic such as ProClin (registered trademark). The salts include those contained for adjustment of ion strength, such as NaCl, as well as those added for the purpose of adjustment of pH of the buffer solution, such as sodium hydroxide. The pH of the sample diluent of the present invention is preferably in the range of 6.0 to 9.0, more preferably 6.5 to 8.0, and further preferably 7.0 to 8.0.

The sample diluent of the present invention is a solution for diluting the sample, and thus, an infant fecal sample is added to the sample diluent for dissolving, extracting, or dispersing the sample for dilution. Thus, typically, the sample and the sample diluent have been mixed when the sample is supplied to a sample pad of a test strip. However, additionally, the detection method of the present invention also includes the case that a sample is supplied to the sample pad directly or after diluted with a diluent other than the sample diluent of the present invention while the sample diluent of the present invention is supplied to the sample pad simultaneously or subsequently.

The sample diluent of the present invention can inhibit nonspecific reaction of an infant fecal sample in immunoassay and is therefore particularly useful as a diluent for an infant fecal sample for immunoassay.

The immunoassay may be any detection method using an immunoreaction and may be, for example, a latex immunoagglutination assay (hereinafter referred to as an LTIA), which is a particle agglutination immunoassay, ELISA, which is a representative labeled immunoassay, or a detection method using immunochromatography, and among them, the detection method using immunochromatography is particularly desirable. When the sample diluent of the present invention is used in LTIA, which is an immunoassay, a fecal sample can be diluted with the sample diluent of the present invention and then mixed with a latex reagent to detect an immunoagglutination reaction. If the latex reagent is made up of a first reagent containing a buffer solution and a second reagent containing a latex reagent, the specimen diluent of the present invention can be used as the first reagent.

The present invention also provides a sample pretreatment method for inhibiting nonspecific reaction of an infant fecal sample, comprising a step of bringing the sample diluent of the present invention into contact with the infant fecal sample.

### (Analyte)

An analyte for the present invention may be any analyte which is contained in infant feces used as a sample and is detectable by using an antigen-antibody reaction, and examples thereof include viruses, parasites, and proteins.

For example, infectious gastroenteritis is mainly caused by viruses and parasites, and examples of viruses defined as the analyte include norovirus, adenovirus, rotavirus, sapovirus, enterovirus, and the like, and examples of parasites defined as the analyte include Cryptosporidium, amebic dysentery, Giardia, and the like.

Additionally, examples of viruses defined as the analyte include an influenza virus, and examples of proteins defined as the analyte include human hemoglobin, a hepatitis B virus antibody, a hepatitis C virus antibody, a human immunodeficiency virus antibody, and the like.

### (Immunochromatographic Test Strip)

An immunochromatographic test strip of the present invention is a test strip for developing an infant fecal sample in an insoluble membrane so as to detect a complex between an analyte in the sample and a conjugate. The test strip has a membrane made up of a porous body including (1) a sample-supply portion, (2) a developing portion, and (3) a detecting portion, and a part of the developing portion retaining in an elutable manner a conjugate containing a first antibody labeled with a labeling substance, while the detecting portion has a second antibody immobilized in a part of the developing portion on the downstream side of the conjugate-retaining part.

A compound containing in the molecule a dicarboxylic acid or a tricarboxylic acid for the present invention may be in a form contained in a sample diluent or may be in a form impregnated in a part of a pad constituting the test strip. For example, it may be a sample pad, a third pad, a conjugate pad, or an insoluble membrane. Alternatively, the compound containing in the molecule a dicarboxylic acid or a tricarboxylic acid for the present invention may be in a form impregnated in a filter chip.

The conjugate is an antibody or antigen which is immunologically reactive with the analyte and is immobilized on a label and, with regard to the presence form of the conjugate, the conjugate may be present in a state of being impregnated in a conjugate pad that is a pad other than the sample pad, the third pad, and the insoluble membrane (type A), may be present as a conjugate part in a portion of the sample pad (type B), or may be present as a separate conjugate reagent to be mixed with a specimen separately from the test strip (type C).

The test strip having the conjugate in the presence form of the type A will hereinafter be described.

The sample pad, the conjugate pad, the third pad, and the insoluble membrane are arranged in this order from upstream to downstream in the flow direction of the sample and are arranged such that upward and downward (upper and lower) layers at least partially overlap with each other. An example of a test strip of such an arrangement is shown in Fig. 1.

When a sample containing an analyte is supplied to the sample pad of such a test strip, the analyte flows through the sample pad to the conjugate pad on the downstream side. In the conjugate pad, the analyte and the conjugate come into contact with each other and pass through the pad while forming a complex (agglutination body). Subsequently, the complex passes through the porous third pad disposed in contact with the lower surface of the conjugate pad and is developed into the insoluble membrane.

Since the insoluble membrane has an antibody or antigen which is immunologically reactive with the analyte and is immobilized on a portion thereof, the complex is bound and immobilized onto this membrane due to an immunoreaction. The immobilized complex is detected by a means detecting absorbance, reflected light, fluorescence, magnetism, etc. derived from the conjugate.

The test strip having the conjugate in the presence form of the type B will then be described.

A difference from the type A test strip is that the sample pad and the conjugate pad are integrated, i.e., that a sample-supply portion and a conjugate part are formed in portions of a sample pad.

The sample-supply portion is a site to which a sample containing an analyte is supplied, while the conjugate part is a site containing the conjugate, and the sample-supply portion is located upstream of the conjugate part.

The test strip having the conjugate in the presence form of the type C will then be described.

The difference from the type A test strip is that the conjugate pad is absent and that the conjugate is present as a separate conjugate reagent. For example, a filter chip may be included that has the conjugate incorporated in a filter. By using such a filter chip and allowing a sample diluent and an analyte to pass therethrough, the conjugate and the analyte are bound to form the complex (aggregate). This complex can be supplied to the same test strip as the type A except the conjugate pad is absent, so as to detect the analyte.

Additionally, a compound containing in the molecule a dicarboxylic acid or a tricarboxylic acid for the present invention can be contained in the conjugate pad or the filter chip.

### (Sample Pad)

The sample pad used in the present invention is a site receiving a sample and absorbs a liquid sample in a state of being molded into a pad, and any substances and forms are available as long as they can allow passage of the liquid and the analyte. Specific examples of materials suitable for the sample pad include, but not limited to, fibrous glass (glass fiber), acrylic fiber, hydrophilic polyethylene material, dry paper, paper pulp, woven fabric, and the like. Preferably, a glass fiber pad is used. The sample pad can also have a function of a conjugate pad described later. The sample pad can also contain a blocking reagent usually used for the purpose of preventing/inhibiting nonspecific reaction (adsorption) in the antibody-immobilized membrane.

Additionally, a compound containing in the molecule a dicarboxylic acid or a tricarboxylic acid for the present invention can be contained in the sample pad.

### (Third Pad)

The third pad is desirably disposed as needed depending on properties etc. of the sample and may be any pad as long as the pad can allow passage of the complex between the analyte in the sample and the conjugate. In the present invention, for the purpose of suppressing clogging of the insoluble membrane or capturing a nonspecific reactant derived from feces, the third pad is desirably a porous member made of polysulfone or cellulose acetate.

The average pore diameter of the porous third pad for the present invention is exemplified as 1 to 100 µm, preferably 5 to 80 µm, more preferably 10 to 60 µm, further preferably 15 to 55 µm, particularly preferably 20 to 50 µm, and most preferably 25 to 45 µm.

This is because a diameter less than 1 µm causes clogging making a specimen flow itself slow, while a diameter greater than 100 µm appears to decrease the function of capturing nonspecific reaction substances.

Additionally, a compound containing in the molecule a dicarboxylic acid or a tricarboxylic acid for the present invention can be contained in the third pad.

### (Insoluble Membrane)

The insoluble membrane used in the present invention has at least one detecting portion on which an antibody or antigen immunologically reactive with the analyte is immobilized. The antibody or antigen immunologically reactive with the analyte can be immobilized onto an insoluble membrane support by a conventionally known method.
In the case of a lateral flow-based immunochromatographic reagent, after a solution containing a predetermined concentration of the antibody or the antigen is prepared, the solution can be applied in a line shape to the insoluble membrane support by using an apparatus etc. having a mechanism capable of moving a nozzle in a horizontal direction while discharging the solution at a constant rate therefrom and is dried for immobilization. The concentration of the antibody or the antigen in the solution is preferably 0.1 to 5 mg/mL, more preferably 0.5 to 2 mg. An amount of the antibody or the antigen immobilized on the insoluble membrane support can be optimized by adjusting a discharge rate from the nozzle of the apparatus in the case of the lateral-flow format, and is preferably 0.5 to 2 µL/cm.

A measurement method using the lateral flow-based immunochromatographic reagent is a measurement method based on a development format in which a sample moves in a direction parallel to the insoluble membrane support due to capillarity (capillary phenomenon).

The solution containing an antibody or an antigen at a predetermined concentration can be prepared by adding the antibody or the antigen to a buffer solution. The type of the buffer solution may be a commonly used buffer solution such as phosphate buffer, Tris buffer, and Good's buffer. The buffer solution preferably has pH in a range of 6.0 to 9.5, more preferably 6.5 to 8.5, further preferably 7.0 to 8.0. The buffer solution may further contain salts such as NaCl, a stabilizer such as sucrose and a preservative, and an antiseptic such as ProClin (registered trademark). The salts include those contained for adjustment of ion strength, such as NaCl, as well as those added for the purpose of adjustment of pH of the buffer solution, such as sodium hydroxide.

After an antibody or an antigen is immobilized on an insoluble membrane, the insoluble membrane can be coated for blocking with a commonly used blocking agent in the form of solution or vapor, except the site in which the antibody or the antigen is immobilized.

A control capture reagent conventionally used for an immunochromatographic reagent may be immobilized on the insoluble membrane. The control capture reagent is a reagent for ensuring the reliability of assay and captures a control reagent contained in the conjugate pad. For example, if labeled keyhole-limpet haemocyanin (hereinafter referred to as KLH) is contained as a control reagent in the conjugate pad, an anti-KLH antibody etc. correspond to the control capture reagent. The position of immobilization of the control capture reagent can appropriately be selected in accordance with design of the assay system.

Additionally, a compound containing in the molecule a dicarboxylic acid or a tricarboxylic acid for the present invention can be contained in the insoluble membrane.

A membrane constituting the insoluble membrane used in the present invention can be a known membrane conventionally used as an insoluble membrane support of an immunochromatographic reagent. For example, the membrane may be made of fibers of polyethylene, polyethylene terephthalate, nylons, glass, polysaccharide such as cellulose and cellulose derivatives, ceramics, and the like. Specifically, the membrane may be glass fiber filter paper, cellulose filter paper, and the like., commercially available from Sartorius, Millipore, Toyo Roshi, Whatman, and the like. Particularly, UniStar CN140 from Sartorius is preferable. By selecting a pore diameter and a structure of the insoluble membrane support as needed, the rate of flow of the complex between the conjugate and the analyte in the sample can be controlled in the insoluble membrane support.

The immunochromatographic test strip is preferably disposed on a solid phase-supporting body such as a plastic adhesive sheet. The solid phase-supporting body is made of a material not hindering the capillary flow of the sample and the conjugate. The immunochromatographic test strip may be fixed to the solid phase-supporting body with an adhesive etc. In this case, an adhesive component etc., are also made of a material not hindering the capillary flow of the sample and the conjugate. The immunochromatographic test strip can be used after stored in or mounted on an appropriate container (housing) with consideration given to the size of the immunochromatographic test strip, the method and position of addition of the sample, the position of formation of the detecting portion on the insoluble membrane support, the signal detection method, etc., and such a stored/mounted state is referred to as a "device".

### (Antibody or Antigen Immunologically Reactive with Analyte)

The antibody or antigen immunologically reactive with the analyte used in the present invention is an antibody or an antigen capable of binding to the analyte and is preferably an antibody when the analyte is a virus or an antigen, or an antigen when the analyte is an antibody. The antibody or antigen immunologically reactive with the analyte is immobilized on the label described later and on the detecting portion. Although the antibody or the antigen immobilized on the label and the antibody or the antigen immobilized on the detecting portion may be the same, the antibody or the antigen immobilized on the label and the antibody or the antigen immobilized on the detecting portion are preferably different.

### (Label)

For the label used in the present invention, a known label conventionally used for an immunochromatographic test strip can be used. For example, the label is preferably metal colloidal particles such as colloidal gold particles and colloidal platinum particles, colored latex particles, magnetic particles, fluorescent particles, and the like, and is particularly preferably colloidal gold particles and colored latex particles.

### (Conjugate)

The conjugate used in the present invention is the label as described above with an antibody or antigen immunologically reactive with the analyte immobilized thereon. When rotavirus, adenovirus, or norovirus is detected as the analyte, preferably, the conjugate is an anti-rotavirus monoclonal antibody, an anti-adenovirus monoclonal antibody, or an anti-norovirus monoclonal antibody immobilized on colloidal gold particles.

Examples of the method of immobilizing the antibody or antigen immunologically reactive with the analyte onto the label include physical adsorption, chemical bonding, etc., and immobilization is typically achieved by physical adsorption.

### (Combined Use with Another Nonspecificity Inhibition Method)

The detection method using the immunochromatographic test strip of the present invention can also be used in combination with another nonspecificity inhibition method depending on the measurement condition and the type of the sample.

By combining with another nonspecificity inhibition method, a detection method can be implemented such that the nonspecificity is more effectively inhibited with respect to fecal samples of infants. For example, another blocking agent preventing nonspecific reaction can be employed at the same time. Another blocking agent may be added to the sample diluent of the present invention or may be contained in the sample pad, the third pad, the insoluble membrane, or the conjugate.

Another blocking agent may be an agent having a blocking effect.

Since the sample diluent of the present invention produces a nonspecific reaction-inhibiting effect on infant fecal samples, a combination with another reagent or another configuration producing a nonspecificity-inhibiting effect on adult fecal samples enables detection with the same type of immunochromatographic test strips regardless of a sample type (whether adult or infant) for the same inspection item.

The other configuration may be a third pad, for example.

### (Kit)

A detection kit using immunochromatography of the present invention may be any kit including the immunochromatographic test strip and the sample diluent. The detection kit may also include other reagents necessary for detection, a test tube, a cotton swab for fecal sampling, an instruction manual, a housing for storing the test strip, etc.

### (Others)

In this description, the meaning of "upstream" or "downstream" is used to mean the upstream side or the downstream side in the flow direction of the sample. Therefore, when the test strip of the present invention has a sample pad, a conjugate pad, a third pad, and an insoluble membrane laminated from the top in a partially overlapping manner, the sample pad is on the most upstream side and the insoluble membrane is on the most downstream side. An end pad may be laminated on the upper side overlapping with a downstream end portion of the insoluble membrane and, in this case, the end pad is on the most downstream side.

### EXAMPLES

### [Example 1] Study on Adding Anionic Substances to Sample Diluent <In the Case of Rotavirus and Adenovirus Measurement Reagent>

A study on anionic substances was performed for searching for a substance capable of inhibiting nonspecific reaction.

### 1. Test material

### (1) Test device

A test device including an immunochromatographic test strip using an anti-rotavirus antibody and an anti-adenovirus antibody was prepared.

Fig. 1 shows a schematic configuration diagram of an immunochromatographic test strip of the present invention.

An insoluble membrane (b) is affixed to a plastic adhesive sheet (a), and a third pad (g), a conjugate pad (d), and a sample pad (e) are sequentially arranged and mounted along with an absorbent pad (f) which is arranged and mounted on the opposite end. The conjugate pad is impregnated with a conjugate of colloidal gold sensitized with an anti-adenovirus monoclonal antibody and a conjugate of colloidal gold sensitized with an anti-rotavirus monoclonal antibody, and the insoluble membrane has an anti-adenovirus monoclonal antibody, an anti-rotavirus monoclonal antibody, and a control reagent immobilized on lines perpendicular to the flow direction.

Each of the pads is laminated and arranged, a portion thereof being brought into contact with the upper and the lower pads. The lines comprising the anti-adenovirus monoclonal antibody and the anti-rotavirus monoclonal antibody are referred to as test lines (c1, c2), and the line comprising the control reagent is referred to as a control line (c3).

The structured item acquired by overlapping the constituent elements in this way was cut by a constant width to produce immunochromatographic test strips. The test strips can each be stored and mounted in a dedicated plastic housing to achieve a form of an immunochromatographic test device (not shown in the figure).

### (2) Sample diluent

A sample diluent was adjusted by adding trisodium citrate dihydrate (three carboxy groups in a molecule), maleic acid (two carboxy groups in a molecule), monosodium L-aspartate (two carboxy groups in a molecule), monosodium L-glutamate (two carboxy groups in a molecule), 1-heptanesulfonic acid sodium salt (compound contained in conventional sample diluent), or sodium trichloroacetate 3 (one carboxy group in a molecule) to a phosphate buffer (pH 7.6) to the additive concentration shown in Table 1. A sample diluent without the additives was also prepared.

### (3) Sample

Feces of rotavirus- and adenovirus-negative infants (three infants less than 40 days old) were used as specimens. In 1.0 mL of the sample diluent prepared at (2) described above, 0.1 mg of each of the specimens was suspended, and the supernatant thereof was used as a sample.

### 2. Test method

Coloring intensity of the test lines after 10 minutes, 20 minutes, and 30 minutes from dropping of 120 µL of the sample onto the test device was evaluated by using a color sample called "color chart".

According to the color chart, the coloring intensity was quantified on a scale of 0 to 4 in increments of 0.25, and extremely weak nonspecific reaction less than 0.25 were further evaluated on the basis of SS (very weak) and S (weak). The strength of nonspecific reaction was evaluated from the evaluation result of coloring intensity.

Evaluation criteria are described under Table 1. Evaluation was performed in the same way in Examples described later.

In Tables 1 to 6, "Rota", "Adeno", and "noro" denote the cases that rotavirus, adenovirus, and norovirus, respectively, are defined as detection items.

### 3. Test results

The results are shown in Table 1. As shown in Table 1, when rotavirus and adenovirus in infant feces were detected by using the sample diluent prepared by adding trisodium citrate dihydrate (three carboxy groups in a molecule), maleic acid (two carboxy groups in a molecule), monosodium L-aspartate (two carboxy groups in a molecule), or monosodium L-glutamate (two carboxy groups in a molecule), a nonspecific reaction-inhibiting effect was observed at any of the additive concentrations or all the concentrations.

In contrast, when the sample diluent prepared by adding sodium trichloroacetate 3 (one carboxy group in a molecule) was used, the nonspecific reaction-inhibiting effect was not observed at any additive concentration. When a conventional sample diluent, i.e., the sample diluent containing 1-heptanesulfonic acid sodium salt, was used, the nonspecific reaction-inhibiting effect was not observed.

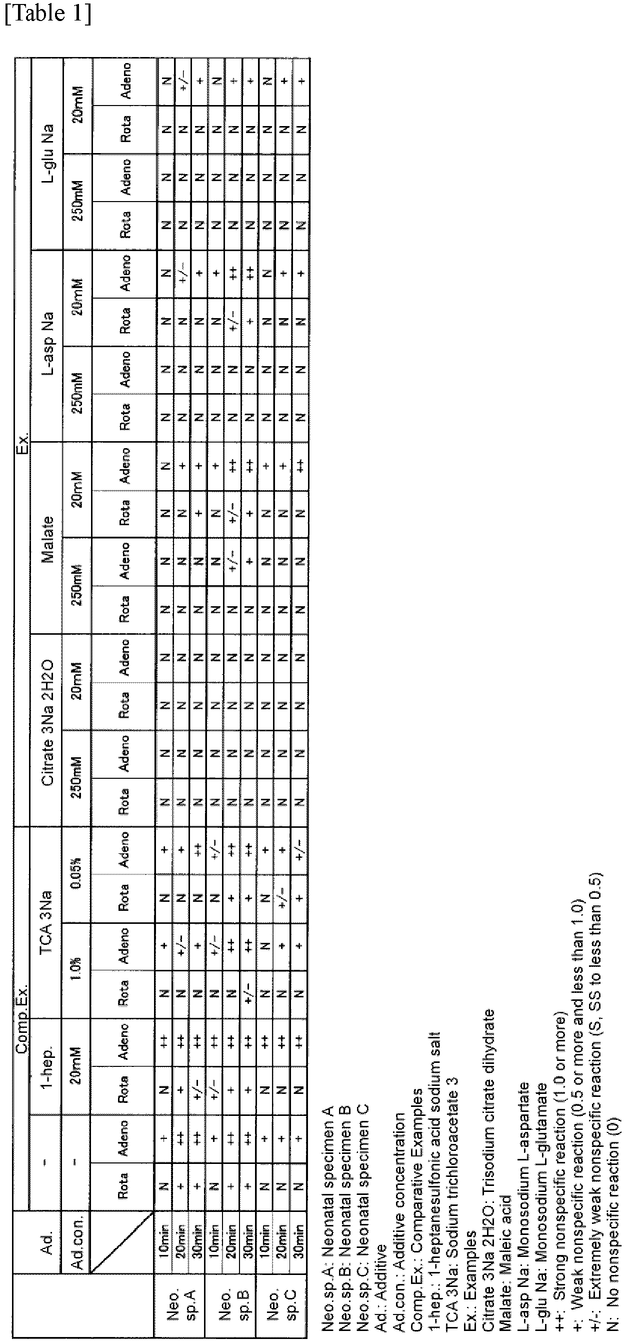

### [Example 2] Studv on Adding Concentration to Sample Diluent of Compound Containing in the Molecule a Dicarboxylic acid or a Tricarboxylic acid <In the Case of Rotavirus and Adenovirus Measurement Reagent>

### 1. Test material

### (1) Test device

Same as Example 1.

### (2) Sample diluent

A sample diluent was adjusted by adding trisodium citrate dihydrate to a phosphate buffer (pH 7.6) to the additive concentration shown in Table 2. A sample diluent without trisodium citrate dihydrate was also prepared.

### (3) Sample

Same as Example 1.

### 2. Test method

Same as Example 1.

### 3. Test results

The results are shown in Table 2. According to Table 2, when the additive concentration of trisodium citrate dihydrate in the sample diluent was in the range of 5 mM to 250 mM, the nonspecific reaction was able to be inhibited for detection of rotavirus and adenovirus as compared to when the trisodium citrate dihydrate was not added. Particularly, although the nonspecific reaction was observed in all the specimens in detection of adenovirus, the nonspecific reaction was able to be completely inhibited by using the sample diluent of the present invention.

**[Table 2]**

| | Ad. | - | | Citrate 3Na 2H2O | | | | | | | | | | | |
|---|---|---|---|---|---|---|---|---|---|---|---|---|---|---|---|
| | Ad.conc. | - | | 5mM | | 10mM | | 20mM | | 50mM | | 100mM | | 250mM | |
| | | Rota | Adeno | Rota | Adeno | Rota | Adeno | Rota | Adeno | Rota | Adeno | Rota | Adeno | Rota | Adeno |
| Neo. sp.A | 10min | N | + | N | N | N | N | N | N | N | N | N | N | N | N |
| | 20min | N | ++ | N | +/- | N | N | N | N | N | N | N | N | N | N |
| | 30min | N | ++ | N | + | N | N | N | N | N | N | N | N | N | N |
| Neo. sp.B | 10min | N | + | N | N | N | N | N | N | N | N | N | N | N | N |
| | 20min | N | ++ | N | + | N | N | N | N | N | N | N | N | N | N |
| | 30min | + | I ++ | + | ++ | + | + | N | N | N | N | N | N | N | N |
| Neo. sp.C | 10min | N | + | N | N | N | N | N | N | N | N | N | N | N | N |
| | 20min | N | ++ | N | + | N | +/- | N | N | N | N | N | N | N | N |
| | 30min | N | ++ | N | + | N | + | N | N | N | N | N | N | N | N |

| | | | | | | | | | | | | | | | |
|---|---|---|---|---|---|---|---|---|---|---|---|---|---|---|---|
| Neo.sp.A: Neonatal specimen A Neo.sp.B: Neonatal specimen B Neo.sp.C: Neonatal specimen C Ad.: Additive Ad.conc.: Additive concentration Citrate 3Na 2H2O: Trisodium citrate dihydrate | | | | | | | | | | | | | | | |

### [Example 3] Study on Adding to Sample Diluent of Compound Containing in the Molecule a Dicarboxylic acid or a Tricarboxylic acid <In the Case of Norovirus Measurement Reagent>

### 1. Test material

### (1) Test device

A test device including an immunochromatographic test strip using an anti-norovirus antibody was prepared.

An insoluble membrane (b) is affixed to a plastic adhesive sheet (a), and a third pad (g), a conjugate pad (d), and a sample pad (e) are sequentially arranged and mounted along with an absorbent pad (f) arranged and mounted on the opposite end. The conjugate pad is impregnated with a conjugate of colloidal gold sensitized with an anti-norovirus monoclonal antibody, and the insoluble membrane has an anti-norovirus monoclonal antibody and a control reagent immobilized on lines perpendicular to the flow direction. Each of the pads is laminated and arranged, a portion thereof being brought into contact with the upper and the lower pads. The line comprising the anti-norovirus monoclonal antibody is referred to as a test line (c1), and the line comprising the control reagent is referred to as a control line (c3).

The structured item acquired by overlapping the constituent elements in this way was cut by a constant width to produce immunochromatographic test strips. The test strips can each be stored and mounted in a dedicated plastic housing to achieve a form of an immunochromatographic test device (not shown in the figure).

### (2) Sample diluent

A sample diluent was adjusted by adding trisodium citrate dihydrate (three carboxy groups in a molecule), maleic acid (two carboxy groups in a molecule), monosodium L-aspartate (two carboxy groups in a molecule), or monosodium L-glutamate (two carboxy groups in a molecule) to a phosphate buffer (pH 7.6) to the additive concentration shown in Table 3. A sample diluent without the additives was also prepared.

### (3) Sample

Feces of norovirus-negative infants (three infants less than 40 days old) were used as specimens. In 1.0 mL of the sample diluent, 0.1 mg of each of the specimens was suspended, and the supernatant thereof was used as a sample.

### 2. Test method

The test method was the same as Example 1 except that the timing of evaluation of the coloring intensity of the test line was set to "after 10, 15, and 30 minutes".

### 3. Test results

The results are shown in Table 3. As shown in Table 3, when norovirus in infant feces was detected by using the sample diluent prepared by adding trisodium citrate dihydrate (three carboxy groups in a molecule), maleic acid (two carboxy groups in a molecule), monosodium L-aspartate (two carboxy groups in a molecule), or monosodium L-glutamate (two carboxy groups in a molecule), a nonspecific reaction-inhibiting effect was observed at any of the additive concentrations or all the concentrations.

**[Table 3]**

| | Ad. | - | Citrate 3Na 2H2O | | Malate | | L-asp Na | | L-glu Na | |
|---|---|---|---|---|---|---|---|---|---|---|
| | Ad.conc. | - | 250mM | 20mM | 250mM. | 20mM | 250mM | 20mM | 250mM | 20mM |
| | | noro | noro | noro | noro | noro | noro | noro | noro | noro |
| Neo. sp.A | 10min | +/- | N | N | N | N | N | N | N | N |
| | 15min | + | N | N | N | +/- | N | N | N | N |
| | 30min | + | N | N | +/- | + | +/- | +/- | N | N |
| Neo. sp.B | 10min | ++ | N | N | +/- | ++ | +/- | + | N | + |
| | 15min | ++ | N | N | + | ++ | +/- | + | N | + |
| | 30min | ++ | +/- | + | + | ++ | N | ++ | N | + |
| Neo. sp.C | 10min | ++ | +/- | N | +/- | ++ | N | + | N | +/- |
| | 15min | ++ | +/- | +/- | + | ++ | N | + | N | + |
| | 30min | ++ | + | + | ++ | ++ | N | + | N | + |

| | | | | | | | | | | |
|---|---|---|---|---|---|---|---|---|---|---|
| Neo.sp.A: Neonatal specimen A Neo.sp.B: Neonatal specimen B Neo.sp.C: Neonatal specimen C Ad.: Additive Ad.conc.: Additive concentration Citrate 3Na 2H2O: Trisodium citrate dihydrate Malate: Maleic acid L-asp Na: Monosodium L-aspartate L-glu Na: Monosodium L-glutamate | | | | | | | | | | |

### [Example 4] Studv on Adding Concentration to Sample Diluent of Compound Containing in the Molecule a Dicarboxylic acid or a Tricarboxylic acid <In the Case of Norovirus Measurement Reagent>

### 1. Test material

### (1) Test device

The same test device as Example 3 was manufactured.

### (2) Sample diluent

A sample diluent was adjusted by adding trisodium citrate dihydrate to a phosphate buffer (pH 7.6) to the additive concentration shown in Table 4. A sample diluent without trisodium citrate dihydrate was also prepared.

### (3) Sample

Feces of norovirus-negative infants (three infants less than 40 days old) were used as specimens. In 1.0 mL of the sample diluent, 0.1 mg of each of the specimens was suspended, and the supernatant thereof was used as a sample.

### 2. Test method

Same as Example 3.

### 3. Test results

The results are shown in Table 4. As shown in Table 4, when the additive concentration of trisodium citrate dihydrate in the sample diluent was in the range of 5 mM to 250 mM, the nonspecific reaction-inhibiting effect was recognized as compared to when the trisodium citrate dihydrate was not added.

**[Table 4]**

| | Ad. | Citrate 3Na 2H2O | | | | | | |
|---|---|---|---|---|---|---|---|---|
| | Ad.conc. | - | 5mM | 10mM | 20mM | 50mM | 100mM | 250mM |
| | | noro | noro | noro | noro | noro | noro | noro |
| Neo. sp.A | 10min | + | N | N | N | N | N | +/- |
| | 15min | ++ | N | N | N | N | N | +/- |
| | 30min | ++ | + | N | N | N | N | +/- |
| Neo. sp.B | 10min | + | + | N | N | N | N | N |
| | 15min | ++ | + | + | N | N | N | N |
| | 30min | ++ | ++ | + | N | N | N | N |
| Neo. sp.C | 10min | ++ | + | + | N | N | N | N |
| | 15min | ++ | ++ | + | N | N | N | N |
| | 30min | ++ | ++ | ++ | N | N | N | N |

| | | | | | | | | |
|---|---|---|---|---|---|---|---|---|
| Neo.sp.A: Neonatal specimen A Neo.sp.B: Neonatal specimen B Neo.sp.C: Neonatal specimen C Ad.: Additive Ad.conc.: Additive concentration Citrate 3Na 2H2O: Trisodium citrate dihydrate | | | | | | | | |

### [Example 5] Study on Adding to Sample Diluent of Compound Containing in the Molecule a Dicarboxylic acid or a Tricarboxylic acid <In the Case of Norovirus Measurement Reagent and Child Specimen>

Examples 1 to 4 are tests using feces of infants (neonates) as specimens, and it was confirmed whether the nonspecific reaction-inhibiting effect of the present invention is observed also for child specimens.

### 1. Test material

### (1) Test device

Same as Example 3.

### (2) Sample diluent

A sample diluent was adjusted by adding trisodium citrate dihydrate to a phosphate buffer (pH 7.6) to the additive concentration shown in Table 5. A sample diluent without trisodium citrate dihydrate was also prepared.

### (3) Sample

Feces of norovirus-negative children (two years old, two children) were used as specimens. In 1.0 mL of the sample diluent, 0.1 mg of each of the specimens was suspended, and the supernatant thereof was used as a sample.

### 2. Test method

The test method was the same as Example 1 except that the timing of evaluation of the coloring intensity of the test line was set to "after 15 minutes".

### 3. Test results

The results are shown in Table 5. As shown in Table 5, it was confirmed that nonspecific reaction can be inhibited also in child specimens by using the specimen diluent of the present invention.

**[Table 5]**

| | Ad. | Citrate 3Na 2H2O | |
|---|---|---|---|
| | Ad.conc. | - | **50mM** |
| | | noro | noro |
| Chi.sp.1 (2yr.) | 15min | + | - |
| Chi.sp.2 (2yr.) | 15min | - | - |

| | | | |
|---|---|---|---|
| Chi.sp1 (2yr.): Child specimen 1 (two years old) Chi.sp.2 (2yr.): Child specimen 2 (two years old) Ad.: Additive Ad.conc.: Additive concentration Citrate 3Na 2H2O: Trisodium citrate dihydrate | | | |

### [Reference Example] Study on Adding to Sample Diluent Compound Containing in the Molecule Two or More Carboxy Groups <In the Case of Norovirus Measurement Reagent and Adult Specimen>

In Examples 1 to 5, feces of infants were used as specimens to confirm the nonspecificity-inhibiting effect of the diluent of the present invention in the immunochromatographic detection method. Therefore, it was investigated whether this nonspecificity-inhibiting effect was also produced for adult specimens.

### 1. Test material

### (1) Test device

Same as in Example 3.

### (2) Sample Diluent

A sample diluent was adjusted by adding trisodium citrate dihydrate to a phosphate buffer (pH 7.6) to the additive concentration shown in Table 6. A sample diluent without trisodium citrate dihydrate was also prepared.

### (3) Sample

Feces of two norovirus-negative adults were used as specimens. In 1.0 mL of the sample diluent, 0.1 mg of each of the specimens was suspended, and the supernatant thereof was used as a sample.

### 2. Test method

The test method was the same as Example 1 except that the timing of evaluation of the coloring intensity of the test line was set to "after 10, 15, and 30 minutes".

### 3. Test results

The results are shown in Table 6. From this result, strong nonspecific reaction was observed in the adult specimens, and the nonspecificity-inhibiting effect of the sample diluent of the present invention was not observed. Therefore, it was considered that the nonspecific reaction-inhibiting effect of the sample diluent of the present invention containing di- or tri- carboxylic acid is an effect of specifically inhibiting nonspecific reaction derived from infant specimens.

**[Table 6]**

| | Ad. | Citrate 3Na 2H2O | | | | | | |
|---|---|---|---|---|---|---|---|---|
| | Ad.conc. | - | 5mM | 10mM | 20mM | 50mM | 100mM | 250mM |
| | | noro | noro | noro | noro | noro | noro | noro |
| Adt.sp.1 | 10min | ++ | ++ | ++ | ++ | ++ | ++ | ++ |
| | 15min | ++ | ++ | ++ | ++ | ++ | ++ | ++ |
| | 30min | ++ | ++ | ++ | ++ | ++ | ++ | ++ |
| Adt.sp.2 | 10min | ++ | ++ | ++ | ++ | ++ | ++ | ++ |
| | 15min | ++ | ++ | ++ | ++ | ++ | ++ | ++ |
| | 30min | ++ | ++ | ++ | ++ | ++ | ++ | ++ |

| | | | | | | | | |
|---|---|---|---|---|---|---|---|---|
| Adt.sp.1 : Adult specimen 1 Adt.sp.2: Adult specimen 1 Ad.: Additive Ad.conc.: Additive concentration Citrate 3Na 2H2O: Trisodium citrate dihydrate | | | | | | | | |

### INDUSTRIAL APPLICABILITY

According to the present invention, an analyte in a sample derived from feces can accurately be detected in an immunochromatographic detection method while inhibiting nonspecific reaction unique to the feces of infants. An analyte in fecal samples of infants can accurately be detected by using immunochromatography because the present invention effectively inhibits nonspecific reaction in the fecal samples of infants.

### REFERENCE SIGNS LIST

(a) plastic adhesive sheet
(b) insoluble membrane
(c1) test line
(c2) test line
(c3) control line
(d) conjugate pad
(e) sample pad
(f) absorbent pad
(9) third pad

## Claims

1. An immunochromatographic method of detecting an analyte in a fecal sample of neonates, infants, and children under seven years old,
(I) comprising the step of:
performing an immunoreaction in the presence of a compound having in the molecule two or three carboxy groups,
or
(II) comprising the steps of:
(A) supplying a sample diluent containing (i) a compound having in the molecule two or three carboxy groups and (ii) a buffer solution as well as a fecal sample to a sample-supply portion of a test strip,
the test strip having
a membrane made up of a porous body including at least the sample-supply portion, a developing portion, and a detecting portion, a part of the developing portion retaining in an elutable manner a conjugate containing a first antibody labeled with a labeling substance, and the detecting portion having a second antibody immobilized in a part of the developing portion on the downstream side of the conjugate-retaining part;
(B) bringing the analyte in the sample into contact with the conjugate; and
(C) detecting a complex between the analyte in the sample and the conjugate in the detecting portion,
wherein said compound having in the molecule two or three carboxy groups is a dicarboxylic acid or a tricarboxylic acid.

2. A method of inhibiting nonspecific reaction in an immunochromatographic method of detecting an analyte in a fecal sample of neonates, infants, and children under seven years old,
(I) comprising the step of:
performing an immunoreaction in the presence of a compound having in the molecule two or three carboxy groups,
or
(II) comprising the steps of:
(A) supplying a sample diluent containing (i) a compound having in the molecule two or three carboxy groups and (ii) a buffer solution as well as a fecal sample to a sample-supply portion of a test strip,
the test strip having a membrane made up of a porous body including at least the sample-supply portion, a developing portion, and a detecting portion, a part of the developing portion retaining in an elutable manner a conjugate containing a first antibody labeled with a labeling substance, the detecting portion having a second antibody immobilized in a part of the developing portion on the downstream side of the conjugate-retaining part;
(B) bringing the analyte in the sample into contact with the conjugate; and
(C) detecting a complex between the analyte in the sample and the conjugate in the detecting portion,
wherein said compound having in the molecule two or three carboxy groups is a dicarboxylic acid or a tricarboxylic acid.

3. Use of an immunochromatographic detection kit for detecting an analyte in a fecal sample of neonates, infants, and children under seven years old, wherein the immunochromatographic detection kit comprises:
(1) an immunochromatographic test strip having a membrane made up of a porous body including at least a sample-supply portion, a developing portion, and a detecting portion, a part of the developing portion retaining in an elutable manner a conjugate containing a first antibody labeled with a labeling substance, the detecting portion having a second antibody immobilized in a part of the developing portion on the downstream side of the conjugate-retaining part; and
(2) a sample diluent containing a compound having in the molecule two or three carboxy groups and a buffer solution,
wherein said compound having in the molecule two or three carboxy groups is a dicarboxylic acid or a tricarboxylic acid.

4. Use of a diluent for detecting an analyte in a fecal sample of neonates, infants, and children under seven years old in an immunoassay, wherein the diluent contains a compound having in the molecule two or three carboxy groups and a buffer solution, wherein said compound having in the molecule two or three carboxy groups is a dicarboxylic acid or a tricarboxylic acid.

5. A sample pretreatment method for inhibiting nonspecific reaction in immunoassay of a fecal sample of neonates, infants, and children under seven years old, comprising the step of bringing the fecal sample of neonates, infants, and children under seven years old into contact with a sample diluent containing (i) a compound having in the molecule two or three carboxy groups and (ii) a buffer solution, wherein said compound having in the molecule two or three carboxy groups is a dicarboxylic acid or a tricarboxylic acid.

## Patentansprüche

1. Immunchromatographisches Verfahren zum Nachweis eines Analyten in einer fäkalen Probe von Neugeborenen, Säuglingen und Kindern unter sieben Jahren,
(I) umfassend den Schritt von:
Durchführung einer Immunreaktion in Gegenwart einer Verbindung mit zwei oder drei Carboxygruppen im Molekül,
oder
(II) umfassend die Schritte von:
(A) Zuführen eines Probenverdünnungsmittels, das (i) eine Verbindung mit zwei oder drei Carboxygruppen im Molekül und (ii) eine Pufferlösung enthält, sowie einer fäkalen Probe zu einem Probenzuführungsabschnitt eines Teststreifens,
wobei der Teststreifen aufweist
eine Membran, die aus einem porösen Körper besteht, der mindestens den Probenzuführabschnitt, einen Entwicklungsabschnitt und einen Nachweisabschnitt einschließt, wobei ein Teil des Entwicklungsabschnitts in eluierbarer Weise ein Konjugat zurückhält, das einen ersten Antikörper enthält, der mit einer Markierungssubstanz markiert ist, und der Nachweisabschnitt einen zweiten Antikörper aufweist, der in einem Teil des Entwicklungsabschnitts auf der downstream gelegenen Seite des Konjugat-zurückhaltenden Teils immobilisiert ist;
(B) Inkontaktbringen des Analyten in der Probe mit dem Konjugat; und
(C) Nachweisen eines Komplexes zwischen dem Analyten in der Probe und dem Konjugat in dem Nachweisteil,
wobei die Verbindung mit zwei oder drei Carboxygruppen im Molekül eine Dicarboxylsäure oder eine Tricarboxylsäure ist.

2. Verfahren zur Inhibierung einer unspezifischen Reaktion in einem immunochromatographischen Verfahren zum Nachweis eines Analyten in einer fäkalen Probe von Neugeborenen, Säuglingen und Kindern unter sieben Jahren,
(I) umfassend den Schritt von:
Durchführen einer Immunreaktion in Gegenwart einer Verbindung mit zwei oder drei Carboxygruppen im Molekül,
oder
(II) umfassend die Schritte von:
(A) Zuführen eines Probenverdünnungsmittels, das (i) eine Verbindung mit zwei oder drei Carboxygruppen im Molekül und (ii) eine Pufferlösung enthält, sowie einer fäkalen Probe zu einem Probenzuführungsabschnitt eines Teststreifens,
wobei der Teststreifen eine Membran aufweist, die aus einem porösen Körper besteht, der mindestens den Probenzuführabschnitt, einen Entwicklungsabschnitt und einen Nachweisabschnitt einschließt, wobei ein Teil des Entwicklungsabschnitts in eluierbarer Weise ein Konjugat zurückhält, das einen ersten Antikörper enthält, der mit einer Markierungssubstanz markiert ist, wobei der Nachweisabschnitt einen zweiten Antikörper aufweist, der in einem Teil des Entwicklungsabschnitts auf der downstream gelegenen Seite des Konjugat-zurückhaltenden Teils immobilisiert ist;
(B) Inkontaktbringen des Analyten in der Probe mit dem Konjugat; und
(C) Nachweisen eines Komplexes zwischen dem Analyten in der Probe und dem Konjugat in dem Nachweisteil,
wobei die Verbindung mit zwei oder drei Carboxygruppen im Molekül eine Dicarboxylsäure oder eine Tricarboxylsäure ist.

3. Verwendung eines immunochromatographischen Nachweiskits zum Nachweis eines Analyten in einer fäkalen Probe von Neugeborenen, Säuglingen und Kindern unter sieben Jahren, wobei das immunochromatographische Nachweiskit umfasst
(1) einen immunchromatographischen Teststreifen mit einer Membran, die aus einem porösen Körper besteht, der mindestens einen Probenzuführungsabschnitt, einen Entwicklungsabschnitt und einen Nachweisabschnitt einschließt, wobei ein Teil des Entwicklungsabschnitts in eluierbarer Weise ein Konjugat zurückhält, das einen ersten Antikörper enthält, der mit einer Markierungssubstanz markiert ist, wobei der Nachweisabschnitt einen zweiten Antikörper aufweist, der in einem Teil des Entwicklungsabschnitts auf der downstream gelegenen Seite des konjugatrückhaltenden Teils immobilisiert ist; und
(2) ein Probenverdünnungsmittel, das eine Verbindung mit zwei oder drei Carboxygruppen im Molekül und eine Pufferlösung enthält,
wobei die Verbindung mit zwei oder drei Carboxygruppen im Molekül eine Dicarboxylsäure oder eine Tricarboxylsäure ist.

4. Verwendung eines Verdünnungsmittels zum Nachweis eines Analyten in einer fäkalen Probe von Neugeborenen, Säuglingen und Kindern unter sieben Jahren in einem Immunoassay, wobei das Verdünnungsmittel eine Verbindung mit zwei oder drei Carboxygruppen im Molekül und eine Pufferlösung enthält, wobei die Verbindung mit zwei oder drei Carboxygruppen im Molekül eine Dicarboxylsäure oder eine Tricarboxylsäure ist.

5. Probenvorbehandlungsverfahren zur Inhibierung einer unspezifischen Reaktion bei einem Immunoassay einer fäkalen Probe von Neugeborenen, Säuglingen und Kindern unter sieben Jahren, umfassend den Schritt des Inkontaktbringens der fäkalen Probe von Neugeborenen, Säuglingen und Kindern unter sieben Jahren mit einem Probenverdünnungsmittel, enthaltend (i) eine Verbindung mit zwei oder drei Carboxygruppen im Molekül und (ii) eine Pufferlösung, wobei die Verbindung mit zwei oder drei Carboxygruppen im Molekül eine Dicarboxylsäure oder eine Tricarboxylsäure ist.

## Revendications

1. Procédé immunochromatographique de détection d'un analyte dans un échantillon fécal de nouveau-nés, de nourrissons et d'enfants de moins de sept ans,
(I) comprenant l'étape de :
la réalisation d'une immunoréaction en présence d'un composé présentant, dans la molécule, deux ou trois groupes carboxy,
ou
(II) comprenant les étapes de :
(A) la fourniture d'un diluant d'échantillon contenant (i) un composé présentant, dans la molécule, deux ou trois groupes carboxy et (ii) une solution tampon ainsi qu'un échantillon fécal à une portion de fourniture d'échantillon d'une bande d'essai,
la bande d'essai présentant
une membrane constituée d'un corps poreux incluant au moins la portion de fourniture d'échantillon, une portion de développement et une portion de détection, une partie de la portion de développement retenant, d'une manière éluable, un conjugué contenant un premier anticorps marqué avec une substance de marquage, et la portion de détection présentant un second anticorps immobilisé dans une partie de la portion de développement sur le côté en aval de la partie de rétention de conjugué ;
(B) la mise de l'analyte dans l'échantillon en contact avec le conjugué ; et
(C) la détection d'un complexe entre l'analyte dans l'échantillon et le conjugué dans la portion de détection,
dans lequel ledit composé présentant, dans la molécule, deux ou trois groupes carboxy est un acide dicarboxylique ou un acide tricarboxylique.

2. Procédé d'inhibition de réaction non spécifique dans un procédé immunochromatographique de détection d'un analyte dans un échantillon fécal de nouveau-nés, de nourrissons et d'enfants de moins de sept ans,
(I) comprenant l'étape de :
la réalisation d'une immunoréaction en présence d'un composé présentant, dans la molécule, deux ou trois groupes carboxy,
ou
(II) comprenant les étapes de :
(A) la fourniture d'un diluant d'échantillon contenant (i) un composé présentant, dans la molécule, deux ou trois groupes carboxy et (ii) une solution tampon ainsi qu'un échantillon fécal à une portion de fourniture d'échantillon d'une bande d'essai,
la bande d'essai présentant une membrane constituée d'un corps poreux incluant au moins la portion de fourniture d'échantillon, une portion de développement et une portion de détection, une partie de la portion de développement retenant, d'une manière éluable, un conjugué contenant un premier anticorps marqué avec une substance de marquage, la portion de détection présentant un second anticorps immobilisé dans une partie de la portion de développement sur le côté en aval de la partie de rétention de conjugué ;
(B) la mise de l'analyte dans l'échantillon en contact avec le conjugué ; et
(C) la détection d'un complexe entre l'analyte dans l'échantillon et le conjugué dans la portion de détection,
dans lequel ledit composé présentant, dans la molécule, deux ou trois groupes carboxy est un acide dicarboxylique ou un acide tricarboxylique.

3. Utilisation d'un kit de détection immunochromatographique pour détecter un analyte dans un échantillon fécal de nouveau-nés, de nourrissons et d'enfants de moins de sept ans, dans lequel le kit de détection immunochromatographique comprend :
(1) une bande d'essai immunochromatographique présentant une membrane constituée d'un corps poreux incluant au moins une portion de fourniture d'échantillon, une portion de développement et une portion de détection, une partie de la portion de développement retenant, d'une manière éluable, un conjugué contenant un premier anticorps marqué avec une substance de marquage, et la portion de détection présentant un second anticorps immobilisé dans une partie de la portion de développement sur le côté en aval de la partie de rétention de conjugué ; et
(2) un diluant d'échantillon contenant un composé présentant, dans la molécule, deux ou trois groupes carboxy et une solution tampon,
dans lequel ledit composé présentant, dans la molécule, deux ou trois groupes carboxy est un acide dicarboxylique ou un acide tricarboxylique.

4. Utilisation d'un diluant pour détecter un analyte dans un échantillon fécal de nouveau-nés, de nourrissons et d'enfants de moins de sept ans dans un dosage immunologique, dans lequel le diluant contient un composé présentant, dans la molécule, deux ou trois groupes carboxy et une solution tampon, dans lequel ledit composé présentant, dans la molécule, deux ou trois groupes carboxy est un acide dicarboxylique ou un acide tricarboxylique.

5. Procédé de prétraitement d'échantillon pour inhiber une réaction non spécifique dans un dosage immunologique d'un échantillon fécal de nouveau-nés, de nourrissons et d'enfants de moins de sept ans, comprenant l'étape de la mise de l'échantillon fécal de nouveau-nés, de nourrissons et d'enfants de moins de sept ans en contact avec un diluant d'échantillon contenant (i) un composé présentant, dans la molécule, deux ou trois groupes carboxy et (ii) une solution tampon, dans lequel ledit composé présentant, dans la molécule, deux ou trois groupes carboxy est un acide dicarboxylique ou un acide tricarboxylique.
